# EUROPEAN PATENT APPLICATION

(11) **EP 1 842 556 A1**
(43) Date of publication of application: **10.10.2007**
(21) Application number: 06711849.7
(22) Date of filing: 18.01.2006
(51) Int. Cl.: A61K 45/00, A61K 31/5377, A61P 25/14, A61P 43/00, C07D 413/12

(54) **THERAPEUTIC AGENT FOR ATTENTION-DEFICIT HYPERACTIVITY DISORDER**

(30) Priority: 18.01.2005 JP 2005010226
(71) Applicant: Mitsubishi Pharma Corporation, Osaka-shi, Osaka 541-0046 (JP)
(72) Inventor: KURIYAMA, Makoto, MITSUBISHI PHARMA CORPORATION, Chuo-ku, Tokyo 1038405 (JP)
(74) Representative: Weber, Thomas
(86) International application number: PCT/JP2006/300580
(87) International publication number: WO 2006/077846

(57) **Abstract**

A prophylactic and/or therapeutic drug for AD/HD, containing teniloxazine or an optical isomer thereof, or a pharmaceutically acceptable salt thereof, or a hydrate thereof, as the active ingredient.

## Description

### Technical Field

The present invention relates to a drug that is useful for the prevention and/or treatment of attention deficit hyperactivity disorder.

### Background Art

Hyperactivity disorder or attention deficit hyperactivity disorder (hereinafter referred to as AD/HD), a behavioral disorder characterized by attention disorder and hyperactivity/impulsiveness, is a disease that is prevalent in children. AD/HD is reported to affect about 5% of all school children in the United States.

Regarding the cause of AD/HD, various causal factors such as perinatal brain disorder, heredity, central norepinephrine or dopamine system abnormality, and lead poisoning have been hypothesized, but the accurate cause remains unknown. This disease develops in children under 6 years old, and the hyperactivity condition as is tends to calm with age. However, because the disease often progresses to oppositional provocative disorder and the like, adequate treatment must be given in early stage.

Conventionally, treatment for this disease mainly employs remedial education methods; psychotherapy, educational therapy, or sensory integrative therapy is performed on patients to help them enjoy appropriate social life. For drug therapy, methylphenidate (marketed as Ritalin by Novartis) and amphetamine (marketed as Adderall), which are psychostimulants/psychotropic drugs, have mainly been used on significant scales, but both of these drugs pose the problem of severe adverse reactions, although they are effective against

Recently, Atomoxetine (chemical name: ((-)-N-methyl-3-((2-methylphenyl)oxy)-3-phenyl-1-aminopropane hydrochloride: marketed as Strattera by Eli Lilly) was launched as a therapeutic drug for AD/HD (see non-patent document 1).

Atomoxetine is a selective noradrenaline re-uptake inhibitor, and has a mechanism of action different from that of existing AD/HD therapeutic drugs. However, how Atomoxetine suppresses the symptoms of AD/HD has not completely been elucidated. Although Atomoxetine is expected to have no habituation, side effects such as vomiting and sedation and influences of long-term use on growth remain unknown.

The only non-psychostimulant as an AD/HD therapeutic drug is Atomoxetine; it cannot be said that the demands in clinical settings are fully met.

On the other hand, teniloxazine (chemical name: (±)-2-[[o-(2-thenyl)phenoxy]methyl]morpholine maleic acid salt) (see patent document 1) is reported to have an ameliorating effect on brain disorder in hypoxia, effect on the central nervous system, hypertensive effect, gastric acid secretion inhibitory effect, local anesthetic effect, or analgesic effect (see non-patent document 2), but nothing has been known to date about the pharmacological effect of an optical isomer thereof, and no report is available on the potential as an AD/HD therapeutic drug.
[Patent document 1] Official Gazette for US Patent No. 4005084
[Non-patent document 1] Expert Opinion of Pharmacotherapy 2003 Jul.; 4.(7): 1165-74.
[Non-patent document 2] Folia Pharmacologica Japonica, Pharmacological study of Y-8894 (Report 5), 89, 145-153, 1987

### Disclosure of the Invention

### Problems to Be Solved by the Invention

It is an object of the present invention to provide a therapeutic drug that enables an effective drug therapy for AD/HD.

### Means for solving the problems

The present inventor has found that an optical isomer of teniloxazine has noradrenaline re-uptake inhibitory effect and serotonin (5-HT)2A receptor antagonist effect in combination, further found that a compound having these effects in combination is useful for the treatment of AD/HD, and completed the present invention.

The present inventor has investigated diligently and as a result found that an optical isomer of a compound represented by formula (1):

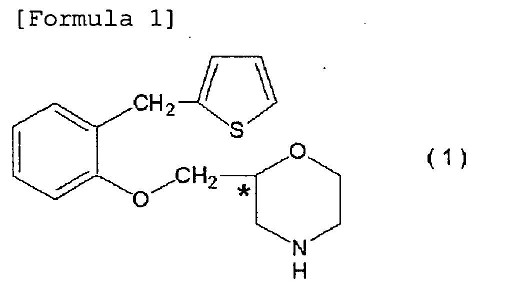

or a pharmaceutically acceptable salt thereof, or a solvate thereof could be a therapeutic drug for AD/HD, and developed the present invention.

Accordingly, the present invention relates to the following (1) to (9).
(1) A method for treating attention deficit hyperactivity disorder, comprising administering a compound or a pharmaceutically acceptable salt thereof, which has mammalian norepinephrine re-uptake inhibitory action and serotonin 2A receptor antagonist action.
(2) A therapeutic drug for attention deficit hyperactivity disorder, comprising a compound or a pharmaceutically acceptable salt thereof, which has mammalian norepinephrine re-uptake inhibitory action and serotonin 2A receptor antagonist action, as the active ingredient.
(3) A pharmaceutical composition for treating attention deficit hyperactivity disorder, comprising a compound or a pharmaceutically acceptable salt thereof, which has mammalian norepinephrine re-uptake inhibitory action and serotonin 2A receptor antagonist action, and a carrier acceptable for pharmaceutical making.
(4) A use of a compound or a pharmaceutically acceptable salt thereof, which has mammalian norepinephrine re-uptake inhibitory action and serotonin 2A receptor antagonist action, for treating attention deficit hyperactivity disorder.
(5) A therapeutic drug for attention deficit hyperactivity disorder, comprising at least one of S-2-[[o-(2-thenyl)phenoxy]methyl]morpholine and R-2-[[o-(2-thenyl)phenoxy]methyl]morpholine, or a pharmaceutically acceptable salt thereof, or a solvate thereof, as the active ingredient.
(6) A therapeutic drug for attention deficit hyperactivity disorder, comprising S-2-[[o-(2-thenyl)phenoxy]methyl]morpholine, or a pharmaceutically acceptable salt thereof, or a solvate thereof, as the active ingredient.
(7) A therapeutic drug for attention deficit hyperactivity disorder, comprising R-2-[[o-(2-thenyl)phenoxy]methyl]morpholine, or a pharmaceutically acceptable salt thereof, or a solvate thereof, as the active ingredient.
(8) A pharmaceutical composition comprising at least one of S-2-[[o-(2-thenyl)phenoxy]methyl]morpholine and R-2-[[o-(2-thenyl)phenoxy]methyl]morpholine, or a pharmaceutically acceptable salt thereof, or a solvate thereof.
(9) The pharmaceutical composition described in 8 above, to be used for the treatment of attention deficit hyperactivity disorder.

### Effect of the Invention

A compound of the formula (1) above, which is the therapeutic drug of the present invention, ameliorates hyperactivity toward its inhibition in 6-OH-DA rats and SHR mice, which are models of AD/HD. With these activities, a compound of the formula (1) above is useful as a prophylactic and/or therapeutic drug for AD/HD. Also, the therapeutic drug of the present invention is of low toxicity and can be safely used as a pharmaceutical.

### Brief Description of the Drawings

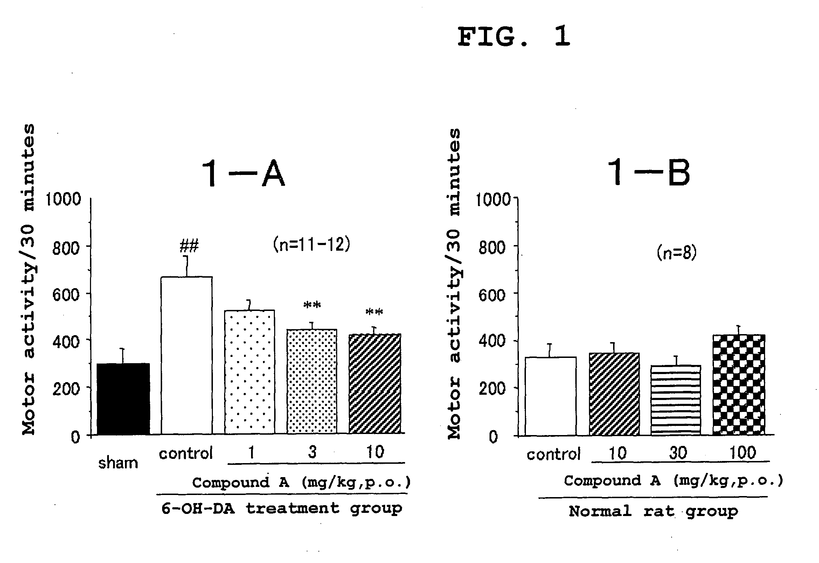

[Figure 1] Figure 1-A is a drawing showing an effect of compound A on increased motor activity and spontaneous activity in rats with juvenile dopamine neulolysis due to 6-OH-DA treatment. Figure 1-B is a drawing showing an effect of compound A on normal rats. ##: indicates that the significance level (P) versus sham is P<0.01 (t-test); **: indicates P<0.01 versus control (Dunnett method).

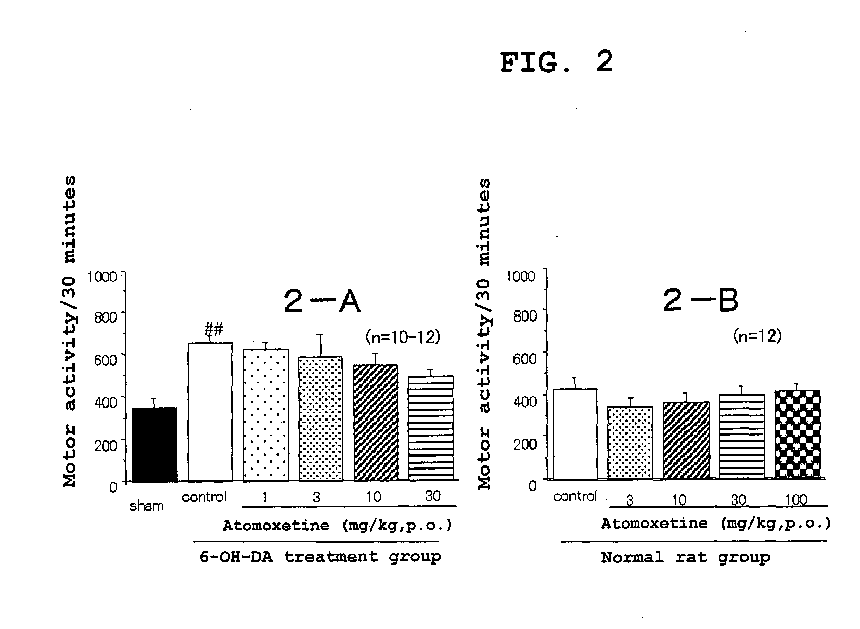

[Figure 2] Figure 2-A is a drawing showing an effect of Atomoxetine on increased motor activity and spontaneous activity in rats with juvenile dopamine neulolysis due to 6-OH-DA treatment. Figure 2-B is a drawing showing an effect of Atomoxetine on normal rats. ##: indicates that the significance level (P) versus sham is P<0.01 (t-test).

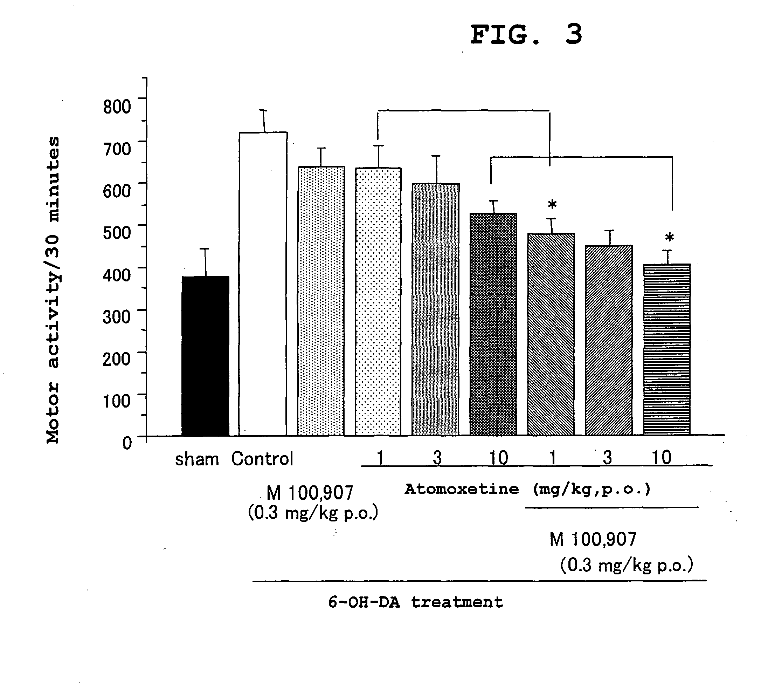

[Figure 3] A drawing showing an effect of a combination of Atomoxetine and M100,907 on increased motor activity and spontaneous activity in rats with juvenile dopamine neulolysis due to 6-OH-DA treatment. * indicates that the significance level (P) for motor activity in the absence of M100,907 is P<0.01 (t-test).

### Best Mode for Embodying the Invention

The present invention is hereinafter described in more detail.
"A compound having mammalian norepinephrine re-uptake inhibitory action and serotonin 2A receptor antagonist action (hereinafter referred to as the active ingredient compound)", used as the active ingredient of the therapeutic drug of the present invention, may be a compound of the formula (1) above in a free form, and a pharmaceutically acceptable acid addition salt or an optionally chosen solvate thereof can also be used. Because the active ingredient compound and pharmaceutically acceptable salt thereof sometimes occur in the form of a solvate, hydrate thereof or other solvates are also encompassed in the method, therapeutic drug, composition, or use of the present invention.

The active ingredient compound can be obtained by synthesizing a racemate by a method described in the Official Gazette for US Patent No. 4005084 and the like, and then optically resolving the racemate using an optical resolution agent (tartaric acid or a derivative thereof, or alanine or a derivative thereof, and the like).

As the pharmaceutically acceptable salt, acid addition salts with inorganic acids (hydrochloric acid, hydrobromic acid, sulfuric acid, phosphoric acid, nitric acid and the like) or organic acids (acetic acid, propionic acid, succinic acid, glycolic acid, lactic acid, malic acid, tartaric acid, citric acid, maleic acid, fumaric acid, methanesulfonic acid, benzenesulfonic acid, p-toluenesulfonic acid, camphorsulfonic acid, ascorbic acid and the like) can be mentioned.

When the therapeutic drug of the present invention is used as a pharmaceutical, a pharmaceutical composition obtained by mixing the active ingredient compound with a carrier acceptable for pharmaceutical making (excipient, binder, disintegrant, taste corrective, odor corrective, emulsifier, diluent, solubilizer and the like) can be orally or parenterally administered in the form of tablets, pills, capsules, granules, powders, syrups, emulsions, elixirs, suspensions, solutions, injections, drip infusions or suppositories and the like that are obtained by preparing the composition according to an ordinary method.

"Parenteral" includes subcutaneous injection, intravenous injection, intramuscular injection, intraperitoneal injection or drip infusion and the like; a preparation for injection, for example, an aqueous suspension or oily suspension for injection, can be prepared using an appropriate dispersing agent or wetting agent and a suspending agent by a method known in the art. The preparation for injection may also be an injectable solution or suspension in solvent that is nontoxic and parenterally administrable. As acceptable useful solvents, water, Ringer's solution, isotonic saline and the like can be mentioned. Furthermore, as a solvent or suspending solvent, a sterile non-volatile oil can also be used in general. To this end, any non-volatile oil and fatty acid can be used, and natural, or synthetic or semi-synthetic fatty oils or fatty acids, and natural or synthetic or semi-synthetic mono- or di-or tri-glycerides are also included.

A suppository for rectal administration can be produced by mixing the active ingredient compound with an appropriate non-irritating filler, for example, one that is solid at normal temperature but liquid at intestinal temperature and melts in the rectum to release a drug, such as cocoa butter and polyethylene glycols.

As solid dosage forms for oral administration, those described above, such as dusts, granules, tablets, pills, and capsules can be mentioned. In such a dosage form, the active ingredient compound can be mixed with at least one additive, for example, sucrose, lactose, cellulose sugar, mannitol, maltitol, dextran, starches, agar, alginates, chitins, chitosans, pectins, tragacanth gums, acacias, gelatins, collagens, casein, albumin, synthetic or semi-synthetic polymers or glycerides. Such a dosage form can also comprise an additional additive as usual; for example, inert diluents, lubricants such as magnesium stearate, preservatives such as parabens, sorbic acid or a salt thereof, antioxidants such as ascorbic acid, α-tocopherol, and cysteine, disintegrants, binders, thickening agents, buffering agents, sweeteners, flavoring agents, perfume agents and the like can be mentioned.

Tablets and pills can also be produced with enteric coating.

As liquids for oral administration, pharmaceutically acceptable emulsion types, syrups, elixirs, suspensions, solutions and the like can be mentioned; they may comprise an inert diluent in common use in the art, for example, water.

A dose is determined according to age, body weight, general health condition, sex, meals, time of administration, method of administration, excretion rate, drug combination, severity of the patient condition being treated at that time, in consideration of them or other factors. The active ingredient compound or a pharmaceutically acceptable salt thereof is of low toxicity and can be safely used, and the daily dose thereof varies depending on the patient condition and body weight, kind of compound, route of administration and the like; for example, it is desirable that the active ingredient compound or a pharmaceutically acceptable salt thereof be administered subcutaneously, intravenously, intramuscularly or intrarectally at about 0.01 to 50 mg/person/day, preferably 0.01 to 20 mg/person/day, for parenteral administration, and at about 0.01 to 150 mg/person/day, preferably 0.1 to 100 mg/person/day, for oral administration.

### Examples

A Preparation Example and the results of a pharmacological test are given below, which, however, are for facilitating the understanding of the present invention and never limit the scope of the invention.

### Reference Example 1: Synthesis of S-2-[[o-(2-thenyl)phenoxy]methyl]morpholine maleic acid salt

58 g of ( 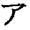 )-2-[[o-(2-thenyl)phenoxy]methyl]morpholine was dissolved in 1 L of ethanol, and while stirring this solution, a solution of 2,3-dihydroxy-N-phenyl-dibutanoic acid monoamide (45 g) in ethanol (400 ml) was added. The solution was concentrated under reduced pressure, isopropyl alcohol was added, and the precipitated crystal was collected by filtration (here, by subjecting the mother liquor deprived of the crystal to the same purification treatment as described below, the stereoisomer in the reverse configuration to that of the title compound can be obtained).

Benzene (600 ml) and an aqueous solution of sodium hydroxide were added to the crystal to separate the solution into layers, and the organic layer was dried, after which the solvent was distilled off. The residue was dissolved in ethanol (500 ml), maleic acid was added, and the solution was concentrated. Isopropyl alcohol was added to the solution, and the precipitated crystal was collected by filtration. The crystal was washed with each of acetone and isopropyl alcohol to yield the title compound (24 g).
Melting point 128-129°C
[α] D=-4.54 (MeOH, C=207.0 mg/10 ml)

### Preparation Formulation Example 1

A general preparation example of an oral agent of the present invention compound is given below.
100 g of S-2-[[o-(2-thenyl)phenoxy]methyl]morpholine maleic acid salt, 200 g of cornstarch, 500 g of lactose, 150 g of carboxymethylcellulose calcium, 75 g of polyvinylpyrrolidone, 75 g of talc, and 250 g of microcrystalline cellulose were blended according to a conventional method, granulated, and compression-molded to produce tablets weighing 120 mg per tablet. Also, desired tablets can be obtained by properly changing the amounts of the compound of the present invention and additives.

The pharmacological action of the pharmaceutical of the present invention is hereinafter described with reference to Experimental Examples.
As the test compounds, (±)-2-[[o-(2-thenyl)phenoxy]methyl]morpholine maleic acid salt, S-2-[[o-(2-thenyl)phenoxy]methyl]morpholine maleic acid salt, and R-2-[[o-(2-thenyl)phenoxy]methyl]morpholine maleic acid salt were used. Hereinafter, these compounds are referred to as teniloxazine and compound A or B, respectively.
Also, Atomoxetine was synthesized by the method described in The Journal of organic chemistry 1988 vol53, No.13:2916-2920 and used; M100,907 ((+)2,3-dimethoxyphenyl-1-[2-4-(piperidine)-methanol]), which is a selective antagonist of serotonin 2A receptor, was synthesized by the method described in European Patent Official Gazette No. 531410, and used in the experiments.

### Experimental Example 1: Suppressive effect on increased motor activity in rats with juvenile dopamine neulolysis due to 6-OH-DA treatment

### [1] Test method

Male SD rats (Charles River Japan) were pre-treated with desipramine (Sigma Ltd.) in accordance with the method of Zhang et al. (Neuropsychopharmacology 2001 Nov;25(5):624-32) on day 5 after birth, after which 6-hydroxydopamine (hereinafter referred to as 6-OH-DA) (Sigma Ltd.) was administered to the cerebral ventricle on one side. During 12 to 18 days after birth, acclimation to a motor activity measurement apparatus (Toyo Sangyo) was performed three times; on day 19 after birth, motor activity was measured and the animals were grouped. On day 20 after birth, compound A (1, 3 or 10 mg/kg, oral) and Atomoxetine (1, 3, 10 or 30 mg/kg, oral) were administered, and motor activity during 30 minutes following 1 hour after administration was measured. For control, 0.5% HPMC (hydroxypropylmethylcellulose) was orally administered. Also, motor activity during 30 minutes of use of Atomoxetine and M100,907 (0.3 mg/kg, oral) in combination was also investigated.

### 2) Results and discussion

By administration of compound A, increased motor activity due to the 6-OH-DA treatment was dose-dependently inhibited, with a significant difference observed from 3 mg/kg (Figure 1-A). Even when normal rats were given compound A, no significant suppression of motor activity was observed (Figure 1-B). On the other hand, by administration of Atomoxetine, increased motor activity due to the 6-OH-DA treatment was dose-dependently suppressed, but no significant suppression was observed (Figure 2-A).
Also, by administering Atomoxetine and M100,907 in combination, increased motor activity due to the 6-OH-DA treatment was remarkably suppressed (Figure 3). This result shows that combining norepinephrine re-uptake inhibitory activity and serotonin 2A receptor antagonist activity is effective in the treatment of AD/HD.

ED₅₀ values for the suppression of increased motor activity due to the 6-OH-DA treatment as 100% were analyzed by linear regression, and determined to be 1.7 and 24 mg/kg for compound A and Atomoxetine, respectively.

### Experimental Example 2: Investigation of affinity of teniloxazine, and compounds A and B for 5-HT2A receptor

### 1) Test method

A specific 5-HT2A receptor binding test was performed in accordance with a publicly known method (Molecular Pharmacology, Vol.21, p.301, 1981).

A crude synaptosome fraction was separated from the cerebral cortex of Wistar rats at 6 to 12 weeks of age (SEAC Yoshitomi), suspended in 50 mmol/L tris-HCl buffer solution (pH 7.7) (Nacalai Tesque, Inc.), and used in the experiments. Next, each test compound at several different concentrations and tritiated ketanserin (final concentration 0.2 nmol/L) (Japan Radioisotope) were added to the synaptosome suspension, and they were reacted at 37°C for 20 minutes. After the reaction, the reaction liquor was suction-filtered through a Whatman GF/B filter (NeuroScience, Inc.), and the filter was washed with 50 mmol/L tris-HCl buffer solution (pH 7.7), after which radioactivity remaining in the filter was measured using a liquid scintillation counter (Aloka Co., Ltd.). Non-specific binding was determined in the presence of 10⁻⁵mmol/L mianserin (Sigma Ltd.). 50% inhibitory concentrations were graphically calculated, and inhibition constants (Ki values) were determined.

### 2) Results and discussion

The Ki values of teniloxazine, and compounds A and B for 5-HT2A receptor were 49, 26, and 1000 nmol/L, respectively; compound A exhibited the highest affinity.

### Experimental Example 3: Investigation of affinity of teniloxazine, and compounds A and B for norepinephrine uptake inhibitory action

### 1) Test method

Using a publicly known method (Journal of Pharmacology and Experimental Therapeutics, Vol.165, p.78, 1969) with partial modification, norepinephrine uptake inhibitory action was measured.

A crude synaptosome fraction was separated from the hyppocampus of a male ddy mouse, suspended in Krebs-Henseleit buffer solution (pH 7.4), and used in the experiments. Next, each test compound at several different concentrations was added to the synaptosome suspension and pre-incubated for 5 minutes, after which tritiated norepinephrine (Japan Radioisotope) was added to the reaction liquor, and they were reacted at 37°C for 10 minutes. After the reaction, the reaction liquor was suction-filtered through a Whatman GF/B filter (NeuroScience, Inc.), and the filter was washed with Krebs-Henseleit buffer solution (pH 7.4), after which radioactivity remaining in the filter was measured using a liquid scintillation counter (Packard company, 3380 model). Norepinephrine concentration dependent uptake was obtained by subtracting the uptake at 0°C from the uptake at 37°C. 50% inhibitory concentrations were graphically calculated, and inhibition constants (Ki values) were determined.

### 2) Results and discussion

The 50% inhibitory concentrations (IC₅₀) of teniloxazine, and compounds A and B for norepinephrine uptake into mouse cerebral synaptosome were 12, 2.6, and 33 nmol/L, respectively; compound A exhibited the highest affinity.

### Industrial Applicability

A compound of the formula (1) above, which is the therapeutic drug of the present invention, ameliorates hyperactivity toward its inhibition in 6-OH-DA rats and SHR mice, which are models of AD/HD. With these activities, a compound of the formula (1) above is useful as a prophylactic and/or therapeutic drug for AD/HD. Also, the therapeutic drug of the present invention is of low toxicity and can be safely used as a pharmaceutical.
This application claims priority based on a patent application No. 2005-010226 filed in Japan.

## Claims

1. A method for treating attention deficit hyperactivity disorder, comprising administering a compound or a pharmaceutically acceptable salt thereof, which has mammalian norepinephrine re-uptake inhibitory action and serotonin 2A receptor antagonist action.

2. A therapeutic drug for attention deficit hyperactivity disorder, comprising a compound or a pharmaceutically acceptable salt thereof, which has mammalian norepinephrine re-uptake inhibitory action and serotonin 2A receptor antagonist action, as the active ingredient.

3. A pharmaceutical composition for treating attention deficit hyperactivity disorder, comprising a compound or a pharmaceutically acceptable salt thereof, which has mammalian norepinephrine re-uptake inhibitory action and serotonin 2A receptor antagonist action, and a carrier acceptable for pharmaceutical making.

4. A use of a compound or a pharmaceutically acceptable salt thereof, which has mammalian norepinephrine re-uptake inhibitory action and serotonin 2A receptor antagonist action, for treating attention deficit hyperactivity disorder.

5. A therapeutic drug for attention deficit hyperactivity disorder, comprising at least one of S-2-[[o-(2-thenyl)phenoxy]methyl]morpholine and R-2-[[o-(2-thenyl)phenoxy]methyl]morpholine, or a pharmaceutically acceptable salt thereof, or a solvate thereof, as the active ingredient.

6. A therapeutic drug for attention deficit hyperactivity disorder, comprising S-2-[[o-(2-thenyl)phenoxy]methyl]morpholine, or a pharmaceutically acceptable salt thereof, or a solvate thereof, as the active ingredient.

7. A therapeutic drug for attention deficit hyperactivity disorder, comprising R-2-[[o-(2-thenyl)phenoxy]methyl]morpholine, or a pharmaceutically acceptable salt thereof, or a solvate thereof, as the active ingredient.

8. A pharmaceutical composition comprising at lease one of S-2-[[o-(2-thenyl)phenoxy]methyl]morpholine and R-2-[[o-(2-thenyl)phenoxy]methyl]morpholine, or a pharmaceutically acceptable salt thereof, or a solvate thereof.

9. The pharmaceutical composition of claim 8, which is to be used for the treatment of attention deficit hyperactivity disorder.
